# FASCICULE DE BREVET EUROPEEN

(11) **EP 3 518 768 B1**
(45) Date de publication et mention de la délivrance du brevet: **24.06.2020**
(21) Numéro de dépôt: 17793699.4
(22) Date de dépôt: 03.10.2017
(51) Int. Cl.: A61B 8/02, A61B 8/08, A61B 8/00

(54) **SYSTÈME ÉLECTRONIQUE DE SURVEILLANCE FOETALE**
ELEKTRONISCHES SYSTEM ZUR FÖTUSÜBERWACHUNG
ELECTRONIC SYSTEM FOR FOETAL MONITORING

(30) Priorité: 03.10.2016 FR 1659535
(43) Date de publication de la demande: 07.08.2019
(73) Titulaire: Nateo Healthcare, 31100 Toulouse (FR)
(72) Inventeur: LANDMAN, Thomas, 31000 Toulouse (FR); BEAUDOIN, Olivier, 31100 Toulouse (FR)
(74) Mandataire: Ipside
(86) Numéro de dépôt international: PCT/FR2017/052712
(87) Numéro de publication internationale: WO 2018/065720

(56) Documents cités:
- WO-A1-2015/082987
- US-A1- 2012 179 046
- US-A1- 2013 123 636

## Description

### DOMAINE TECHNIQUE DE L'INVENTION

La présente invention concerne un système électronique de surveillance fœtale. Elle s'applique, en particulier, au suivi soit antepartum d'une grossesse soit durant l'accouchement en per-partum.

### ETAT DE LA TECHNIQUE

Les systèmes de tococardiographie, appelés pour la suite CTG (initiales de CardioTocoGraphie), comportent plusieurs types de capteurs mesurant plusieurs paramètres diagnostiques biophysiques maternels ou fœtaux. Les capteurs externes non invasifs sont de deux types :
- le tocodynamomètre qui mesure l'activité et les contractions utérines,
- le capteur Doppler ultrasonore qui permet de mesurer le rythme cardiaque fœtal.

Le capteur ultrasonore est posé sur l'abdomen maternel, et sa position doit être optimisée par le personnel de santé. Une ceinture élastique entourant l'abdomen maternel permet le maintien du capteur à une place fixe et permet de garantir un bon couplage.

Certains documents de l'art antérieur proposent également des systèmes de tococardiographie. On connaît par exemple le document de publication US2016120500 qui permet de mesurer la fréquence cardiaque fœtale comprenant un capteur ultrasonore conçu pour réaliser des mesures de fréquence cardiaque fœtale lorsqu'il est fixé à l'abdomen d'une femme enceinte.

Toutefois, un mauvais positionnement du capteur ultrasonore entraîne de fausses mesures et par conséquent de mauvais diagnostics.

D'autres documents sont également connus, comme le document WO2015/082987, US2012/179046 et US2013/123636. Toutefois ces documents n'optimisent pas le positionnement ou le traitement des capteurs ultrasonores.

Si le positionnement ou si l'angulation du capteur ultrasonore n'observent pas des règles précises, des risques de mauvaises mesures peuvent survenir. Sur les CTG de l'état de l'art, l'optimisation du positionnement et de l'orientation du capteur ultrasonore Doppler s'effectue d'abord de manière grossière par une manœuvre de Léopold pour localiser le dos fœtal par exemple.

Les manœuvres de Léopold sont quatre manœuvres classiques employées pour déterminer la position du fœtus dans l'utérus.
- Léopold A : Une ou deux mains sont placées sur le fond utérin et la partie fœtale ressentie est identifiée.
- Léopold B : La surface palmaire d'une main est utilisée pour localiser le dos du fœtus tandis que l'autre main ressent les irrégularités, comme les mains et les pieds.
- Léopold C : Le pouce et troisième doigt sont utilisés pour saisir et déterminer la partie fœtale présentée au niveau de la symphyse pubienne.
- Léopold D : Les deux mains sont utilisées pour décrire la tête fœtale.

Ensuite, dans l'optimisation du positionnement et de l'orientation du capteur ultrasonore, on utilise de manière fine l'audition du son Doppler émis par le moniteur audio du CTG. Le rendu Doppler audio doit être le plus puissant et clair possible.

Un mauvais positionnement du capteur Doppler peut avoir diverses conséquences négatives. Cela peut d'abord entraîner une confusion du rythme fœtal avec le rythme cardiaque maternel et une sous-estimation du RCF (initiales de Rythme Cardiaque Fœtal). Le déplacement du fœtus ou le mouvement de la mère peut aussi nécessiter un repositionnement du capteur et de la ceinture élastique. Une intervention du personnel de santé est alors nécessaire. Cela peut générer des problèmes organisationnels, lorsque plusieurs patientes sont monitorées simultanément à des emplacements différents. Il faut ajouter que l'optimisation du positionnement et de l'orientation du capteur ultrasonore est une procédure nécessitant l'action d'un personnel disposant d'une formation conséquente.

### OBJET DE L'INVENTION

La présente invention vise à remédier à ces inconvénients.

A cet effet, selon un premier aspect, la présente invention vise un système électronique de surveillance fœtale, remarquable en ce qu'il comprend :
- au moins quatre capteurs ultrasonores émettant et réceptionnant des ondes ultrasonores soit de manière simultanée, soit simultanée par groupes, ou soit séquentiellement, les capteurs ultrasonores étant prévus pour être positionnés sur au moins une partie abdominale maternelle pour couvrir la structure cardiaque fœtale en mouvement et réaliser des mesures de fréquence cardiaque fœtale, la distance entre deux capteurs ultrasonores est maintenue par une liaison, semi-rigide ou semi-élastique, afin de préserver la distribution géométrique des capteurs entre eux, la longueur de ladite liaison est comprise entre 30 et 60mm, de préférence entre 37 et 45 mm, le nombre de capteurs ultrasonores (20) forment ensemble un réseau triangulaire, carré, rectangulaire ou circulaire ;
- un module de pilotage des capteurs ultrasonores comprenant une information de commande des ondes ultrasonores envoyées à au moins un capteur ultrasonore,
- un module de traitement des signaux ultrasonores pour estimer le rythme cardiaque fœtal et les mouvements fœtaux à partir du ou des signaux reçus des capteurs ultrasonores.

Grâce à ces dispositions, l'utilisation de plusieurs capteurs ultrasonores permet d'améliorer les mesures. Chaque capteur ultrasonore comporte un transducteur. Le meilleur transducteur pour la mesure du RCF est celui qui crée un faisceau ultrasonore dans l'axe du transducteur qui est le plus aligné avec le mouvement des cavités cardiaques et qui intersecte le plus perpendiculairement possible les interfaces mobiles des structures cardiaques fœtales. De plus, la distance entre la surface de ce capteur optimal et le cœur fœtal doit être la plus courte possible, de manière à minimiser l'atténuation du faisceau ultrason dans les tissus et à garantir la meilleure qualité du signal Doppler.

Grâce à ces dispositions, l'utilisation d'un module de traitement comprenant une information de commande des ondes ultrasonores permet l'utilisation de différents modes de fonctionnement, non limitatifs tels que :
- un mode ultrasonore d'évaluation du volume de liquide amniotique ;
- un mode de détection du rythme cardiaque foetal ;
- un mode de détection des mouvements fœtaux.

Grâce à ces dispositions, le système ne nécessite pas d'optimisation manuelle du placement des capteurs ultrasonores. Le système détermine automatiquement le positionnement du cœur fœtal et modifie sa séquence de tirs suivant les caractéristiques des signaux ultrasonores reçus.

Avantageusement, le système permet de sonder en profondeur des tissus maternels et fœtaux qui sont adjacents au cœur fœtal, suivant les faisceaux ultrasonores générés dans l'abdomen maternel. Ces sondages permettent l'estimation de dimensions de structures d'intérêts pour le diagnostic telle que les dimensions de la cavité amniotique.

Un autre avantage est de permettre de suivre durant le temps de l'examen la position du cœur fœtal ou d'autres organes fœtaux. Durant le suivi peripartum une estimation du déplacement du fœtus est possible pour suivre le déroulement de la descente du l'enfant dans le bassin maternel. En antepartum, une estimation des mouvements fœtaux (du corps fœtal, des membres inférieurs, ou bien des membres supérieurs fœtaux) est menée par le module de traitement des signaux ultrasonores. On sait depuis la publication [Manning FA, Platt LD, Sipos L., Antepartum fetal évaluation: development of a fetal biophysical profile score, Am J Obstet Gynecol. 1980 Mar 15;136(6):787-95] qu'une quantification de ces mouvements permet de s'assurer du bien-être du fœtus.

Un avantage substantiel de l'automatisation de la détection du cœur fœtal ainsi que de la présence d'une population de capteurs est de permettre de diminuer voire d'éliminer les repositionnements de la ceinture et du capteur ultrasonore durant le monitoring. Si le fœtus bouge et quelle que soit sa position, les capteurs n'ont pas à être déplacés. Les effets sont de réduire les risques de mauvaises mesures et d'erreur, de diminuer le temps d'intervention de l'opérateur. Un autre effet est de permettre de confier le positionnement du dispositif diagnostique à un personnel avec moins de formation, ou à la femme enceinte directement.

L'invention est avantageusement mise en œuvre selon les modes de réalisation et les variantes exposées ci-après, lesquelles sont à considérer individuellement ou selon toute combinaison techniquement opérante.

Dans un mode de réalisation, le nombre de capteurs ultrasonores est compris entre 4 et 64, de préférence entre 24 et 32.

Grâce à ces dispositions, les capteurs ultrasonores forment un réseau.

Dans un mode de réalisation, les capteurs ultrasonores forment ensemble un réseau triangulaire, carré, rectangulaire ou circulaire.

Grâce à ces dispositions, les capteurs couvrent une majorité des cas pour permettre de bien détecter le rythme cardiaque fœtal.

La liaison semi-rigide ou semi-élastique comporte une certaine élasticité qui permet la torsion et la flexion du matériau de la liaison. Ainsi les mouvements sont possibles tout en laissant un débattement limité afin de conserver la rigidité géométrique de l'ensemble. La distance entre deux capteurs reste toujours la même quelle que soit la position de la ceinture sur l'abdomen de la femme enceinte, le volume investigué par tous les capteurs est donc le plus grand possible. Ainsi, les faisceaux ultrasonores créés par les transducteurs ne se chevauchent pas. Les faisceaux ultrasonores créés individuellement sont indépendants et séparés.

Dans un mode de réalisation, l'information de commande est un signal dont l'une de ces caractéristiques est prédéterminée par au moins l'un des éléments sélectionnés parmi : la phase, l'énergie, l'amplitude, la fréquence et la forme d'onde.

Dans un mode de réalisation, l'information de commande est indépendante d'un capteur ultrasonore à un autre et en temps réel.

Dans un mode de réalisation, le module de traitement est relié par un élément de communication du dit système, l'élément de communication étant filaire ou sans fil.

Dans un mode de réalisation, la liaison sans fil utilise au moins une des modalités suivantes : les ondes hertziennes, par exemple UHF (initiales de Ultra Haute Fréquence), lumineuses, par exemple infrarouges, sonores, par exemple infrasonores ou ultrasonores et/ou des spécifications de communication sur un réseau, par exemple Bluetooth (marque déposée), Wi-Fi (marque déposée) ou ZigBee (marque déposée).

Dans un mode de réalisation, le système comporte un terminal communiquant configuré pour lire ou traiter les données du système.

Dans un mode de réalisation, le système comporte l'un des éléments sélectionnés parmi : un tocodynamomètre, un capteur de saturation pulsée en oxygène (Sp02), un électrocardiogramme, un thermomètre, au moins un microphone, un accéléromètre, ou un électromyogramme. Le tocodynamomètre est l'instrument non invasif qui permet d'évaluer la force des contractions utérines pendant le travail.

### BREVE DESCRIPTION DES FIGURES

D'autres avantages, buts et caractéristiques de la présente invention ressortent de la description qui suit faite, dans un but explicatif et nullement limitatif, en regard des dessins annexés, dans lesquels :
- la figure 1 représente un schéma de l'application de plusieurs capteurs ultrasonores sur un abdomen maternel selon un mode de réalisation particulier du système objet de la présente invention ;
- les figures 2 à 7 représentent différentes formes de liaison entre des capteurs ultrasonores ;
- la figure 8 représente une réalisation d'une topologie triangulaire d'un réseau de capteurs ultrasonores ;
- la figure 9 représente une réalisation d'architecture du système .

### DESCRIPTION D'EXEMPLES DE REALISATION DE L'INVENTION

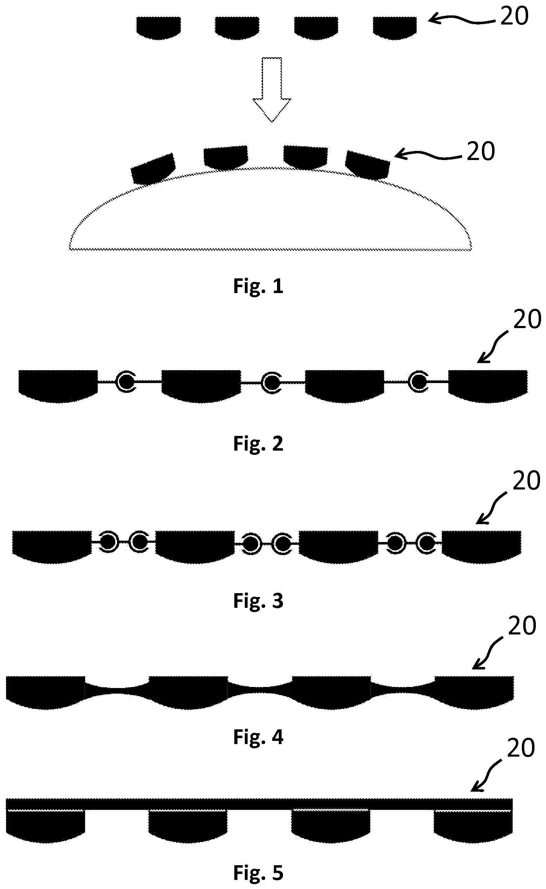

La figure 1 représente quatre capteurs ultrasonores 20 prévus pour être positionnés sur au moins une partie abdominale maternelle pour couvrir la structure cardiaque fœtale en mouvement.

Les figures 2 à 7 représentent différentes formes de liaison entre des capteurs ultrasonores 20. La liaison entre chaque capteur ultrasonore permet de créer une ceinture de capteurs ultrasonores.

Un système spécifique de maintien d'une multiplicité de capteurs est nécessaire afin de préserver la distribution géométrique des capteurs entre eux et aussi d'assurer que l'ensemble des capteurs forme un ensemble conformable ajustable à la morphologie de la patiente. Pour assurer une mesure fiable et performante tout au long de l'examen, il est nécessaire d'assurer un bon couplage acoustique entre chaque capteur et la peau de la patiente (une séquence ultrasons spécifiques peut-être disponible pour vérifier la qualité de ce couplage).

Dans un exemple de réalisation, ce couplage est renforcé à l'aide d'un gel, d'une pâte ou une crème assurant une bonne transmission des ultrasons. Le couplage ne doit pas non plus être perturbé par les mouvements de la patiente ou encore par les contractions. Le système de maintien permet le repositionnement aisé de l'ensemble si besoin.

La distance entre deux capteurs ultrasonores est maintenue par une liaison.

La liaison garde quelle que soit la courbure et la surface sur laquelle elle est positionnée la répartition géométrique des capteurs ultrasonores ou réseau.

Il est présenté six liaisons aux figures 2 à 7 qui permettent d'assurer l'ensemble des fonctionnalités nécessaires soit par des liaisons semi-rigides entre les capteurs ultrasonores (figures 2 et 3) soit par des liaisons semi-élastiques (figures 4 à 7).

Aux figures 2 et 3, chaque capteur ultrasonore est connecté à ses plus proches voisins via une interconnexion mécanique en liaison rotule. L'articulation se trouve physiquement sur les deux capteurs ultrasonores en liaison ou encore sur le bras les reliant l'un à l'autre. Dans une variante, ces liaisons rotules ont un degré de rotation dans les plans perpendiculaires à la surface de contact des capteurs. Dans une autre variante, ces liaisons rotules ont deux degrés de rotation dans les plans perpendiculaires à la surface de contact des capteurs.

Le degré de rotation qui se trouve parallèle à la surface est quant à lui bloqué afin de garder la rigidité géométrique de la distribution de capteur.

La figure 4 utilise pour relier chaque capteur ultrasonore à ses plus proches voisins un lien semi-élastique avec un matériau de type silicone, polyuréthane ou encore élastomère. La rigidité, l'élasticité, la torsion et la flexion du matériau est adaptée afin que les mouvements soient possibles mais avec des débattements limités afin de conserver la rigidité géométrique de l'ensemble.

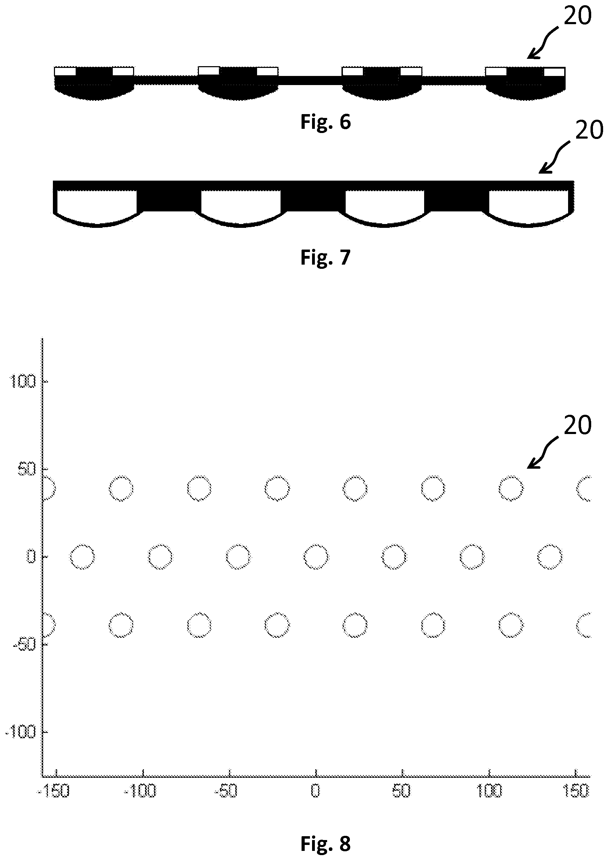

Aux figures 5 et 6, un substrat de type matériau silicone, polyuréthane ou encore élastomère est utilisé afin de maintenir la rigidité géométrique de l'ensemble et la flexibilité nécessaire afin que les capteurs puissent se conformer à la surface de contact.

A la figure 5, les capteurs ultrasonores sont assemblés par collage ou fixation mécanique à la surface du substrat.

A la figure 6, chaque capteur ultrasonore est positionné de manière traversante dans des fenêtres réalisées dans le substrat.

A la figure 7, après avoir positionné mécaniquement les capteurs ultrasonores les uns par rapport aux autres, l'ensemble des capteurs ultrasonores est encapsulé par moulage, injection et collage dans un substrat de type matériau silicone, polyuréthane ou encore élastomère. Le substrat dans cette configuration a des propriétés acoustiques favorisant la propagation des ultrasons ainsi que des propriétés de biocompatibilité du fait que c'est le matériau en interface avec la patiente.

La figure 8 représente une réalisation d'une topologie triangulaire d'un réseau de capteurs ultrasonores 20.

Dans cet exemple, le réseau est triangulaire équilatéral. Une direction principale est la direction latérale-médiale (LM) (abscisse en mm) l'autre direction est la direction cranio-caudale (CC) (ordonnée en mm). Une réalisation typique est un réseau de 8 x 3 capteurs (LM x CC) d'un pas de 37 mm. Son avantage est de laisser des volumes non investigués plus petits à nombre de transducteurs constants.

Dans un autre exemple, le réseau est rectangulaire ou carré régulier, de directions principales : la direction latérale-médiale et la direction cranio-caudale. Le pas du réseau est dicté par le nombre de capteurs et par la taille des capteurs. Une réalisation typique correspond à un réseau carré de pas 45 mm avec 9 x 3 transducteurs (LM x CC).

Dans un autre exemple, le réseau est non régulier, de manière à couvrir plus densément les zones probables de positionnement du ou des fœtus et de leur cœur. Selon un exemple, une réalisation consiste à densifier le réseau triangulaire sous le nombril et au-dessus du nombril et d'écarter les distances inter-transducteur lorsque l'on s'éloigne du nombril dans la direction LM.

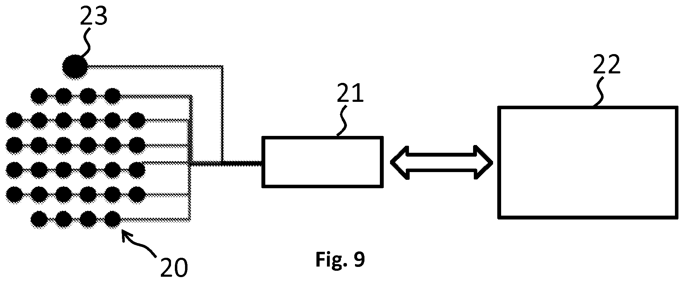

La figure 9 représente une réalisation d'architecture du système.

Le module de pilotage 21 est relié ou connecté sans fil au module de traitement 22.

Le système de surveillance fœtal électronique est composé d'une collection de capteurs ultrasonores 20, d'un tocomètre 23 afin de suivre les contractions utérines.

Dans un autre exemple de réalisation, d'autres capteurs complètent ou remplacent le tocomètre 23, comme par exemple : un capteur de saturation pulsée en oxygène (Sp02), un électrocardiogramme, un thermomètre, au moins un microphone, un accéléromètre, ou un électromyogramme.

Le module de traitement 22 comporte une électronique de pilotage, de conditionnement des signaux ultrasonores et des autres signaux venant des autres capteurs, une unité de traitement des données, un élément de stockage des données et un élément de communication des données.

Dans une réalisation, le module de pilotage 21 et le module de traitement 22 sont alimentés par une batterie lithium-ion, ou une batterie lithium-polymère.

Le module de traitement 22 pour le traitement numérique des données est soit intégré partiellement ou complètement au module de pilotage 21, soit intégré partiellement ou complètement dans un terminal communiquant.

Le terminal communiquant est, par exemple :
- Une tablette numérique,
- Un téléphone mobile, notamment de type « smartphone »,
- Une montre connectée,
- Une télécommande,
- Un ordinateur,
- Une télévision connectée, ou
- Une box internet.

Ce système permet de surveiller la grossesse à distance lorsque les données sont envoyées à un centre professionnel de contrôle et de téléassistance. Ainsi, ce système constitue une solution de télésurveillance de grossesses à risque à domicile ou bien de télédiagnostic dans des zones de désert médical pour lequel la patiente est autonome.

L'électronique du module de pilotage 21 est capable de piloter les X capteurs (dans un exemple, X=32) afin de réaliser les modes ultrasons conventionnels. Le module de pilotage 21 possède Y voies actives (dans un exemple, ici Y=8), chaque voie active possède un émetteur permettant d'exciter les capteurs ultrasonores, c'est l'information de commande des ondes ultrasonores. Ces émetteurs peuvent être pilotés de manière indépendante (en phase, énergie, amplitude, fréquence et forme d'onde) par le module de pilotage 21. Afin d'adresser les Y voies du système aux X capteurs un étage de multiplexage permet d'adresser en temps réel une voie Y à un capteur X de manière arbitraire. Le module de pilotage 21 accepte de piloter les Y voies en toute indépendance. Afin de protéger les étages de réception du module de pilotage 21 des interrupteurs ou switch en terme anglophone Tx/Rx sont positionnés en amont des voies de réception afin de protéger les étages d'entrée du module de pilotage 21. Les étages de réception du module de pilotage 21 sont composés d'un adaptateur d'impédance, un amplificateur linéaire, un amplificateur à gain variable (pour la compensation de l'atténuation ultrasonore du milieu), un étage de filtre analogique et un étage de conversion analogique-numérique (ADC).

L'utilisation de plusieurs capteurs ultrasonores permet d'avoir comme avantage la possibilité d'exciter les transducteurs du réseau suivant des séquences de transmissions et de réceptions ultrasonores spatio-temporelles différentes.

Dans une réalisation du module de pilotage, l'émetteur est une source de tension créant une forme d'onde d'amplitude crête-crête de l'ordre de 5 à 30 V, de durée de 2 à 20 microsecondes et de fréquence centrale de 1 à 4 MHz ; cette forme d'onde est répétée à une période de 0,1 ms à 10 ms. Cette tension excite Y capteurs parmi les X capteurs disponibles par multiplexage.

Dans une réalisation du module, la liste des Y capteurs excités est changée à chaque tir. Le changement en fonction du temps de cette liste constitue la séquence d'excitation des transducteurs.

Dans une réalisation de l'étage de réception du module de pilotage, l'étage de conversion analogique-numérique est suivie d'une démodulation numérique. Ce signal démodulé constitue le signal Doppler ultrasonore complexe.

Dans une réalisation de l'étage de réception, les signaux Doppler venant des Y capteurs sont échantillonnés en Nz = 1 à 20 points (correspondant à des profondeurs différentes) à un taux de répétition correspondant à la répétition des tirs pour former Y ^{∗} Nz (ici 8 à 160) signaux complexes variant dans le temps.

Lorsque la séquence choisie entraine qu'un capteur est périodiquement visité à un intervalle de temps multiple de la période de tirs de l'émetteur, alors la dynamique temporelle des Nz signaux est étroitement reliée aux déplacements du tissu localisé dans le faisceau dudit capteur à la profondeur corresponds à chacun des Nz profondeurs sondées.

Les exemples suivants décrivent des séquences d'excitation :

### 1. Vérification de la bonne connectivité du système

Une séquence simple de tirs ultrasonores test est acquise, comme par exemple en tirant avec les X transducteurs ultrasonores l'un après l'autre. Les données reçues sont testées (comme par exemple leur puissance) pour vérifier la bonne connectivité des composants du système. Si la connectivité des composants n'est pas satisfaisante, un message à l'utilisateur informe d'un problème éventuel de fonctionnement du dispositif.

### 2. Détection du bon contact entre les capteurs et la peau

Une séquence est répétée sur chacun des capteurs. Un indicateur de qualité du signal est mesuré en fonction du capteur. Cet indicateur permet de cartographier la bonne qualité du signal et de détecter si un contact peau/capteur est déficient.

### 3. Séquence de recherche du cœur au travers le réseau de capteurs et en profondeur

Tous les capteurs sont utilisés à la suite sur quelques cycles cardiaques, en utilisant des impulsions ultrasonores basses fréquences et longues et des temps d'écoute longs pour permettre une mesure en profondeur et spatialisée de la puissance Doppler. Le signal Doppler provenant de chaque point d'un faisceau ultrasonore correspond au signal démodulé rétrodiffusé par ce point, filtré dans le temps par un filtre passe-haut dont une fréquence de coupure typique est de 90 Hz. Sa fréquence moyenne instantanée est proportionnelle à la vitesse particulaire axiale du tissu situé à la position d'intérêt. Sa puissance (dite Doppler) est liée au nombre de particules mobiles et réfléchissantes présentes dans le voisinage du point d'intérêt.

La position du cœur fœtal correspondra à la position spatiale qui renvoie une puissance Doppler avec les puissances les plus fortes, avec les dimensions et la périodicité qui tombent dans des intervalles plausibles suivant les informations fœtales. Lorsque l'invention est utilisée pour suivre une grossesse multiple, les positions des N différents fœtus (pour N inférieure ou égale à 8) sont estimées en détectant les positions des N plus puissants signaux Doppler rétrodiffusés par les tissus avec dimensions et périodicité qui sont plausibles avec les informations fœtales.

### 4. Optimisation et boucle de rétroaction pour repositionnement ceinture

Les séquences 2 et 3 peuvent être combinées pour donner des informations de rétroaction à l'utilisateur pour optimiser le positionnement.

### 5. Monitorinq cardiaque avec sous populations de capteurs (les plus proches)

La séquence 3 peut être utilisée avec un sous-groupe de capteurs (typiquement les Y=7 ou 8 capteurs proches de la position initiale du cœur fœtal) pour monitorer le cœur pendant quelques minutes. Dans une réalisation, ces sous-groupes de capteurs correspondent à des capteurs voisins d'un capteur central que l'on inclut dans le sous-groupe. Le sous-groupe choisi en priorité est celui dont le capteur central est le capteur mesure la puissance Doppler maximale. Un avantage par rapport à l'utilisation simultanée de tous les capteurs (Y=X) est par exemple de réduire la consommation électrique ou l'exposition aux ondes ultrasonores.

Dans cette configuration, le rythme cardiaque fœtal est estimé par la méthode suivante à partir des Nz signaux complexes provenant du capteur central observés sur un temps de l'ordre de 1 à 5 secondes.

Dans un premier temps, ces signaux sont filtrés par un filtre passe-haut de fréquence de coupure de l'ordre de 90 Hz. Tous les signaux résultants ou une partie des signaux résultants (signaux Doppler) sont ensuite traités de manière à extraire en fonction du temps leur fréquence instantanée. Cette fréquence Doppler instantanée est directement proportionnelle à la vitesse projetée suivant la direction du faisceau des particules présentes dans le faisceau ultrasonore relatif au capteur central et à sa profondeur investiguée correspondante.

La sélection du nombre de profondeurs utiles à l'évaluation de vitesse axiale du tissu est, par exemple, effectué suivant la puissance moyenne des signaux Doppler.

Lorsque ces particules sont soumises à un mouvement périodique, tel que celui résultant du battement du cœur fœtal, alors le signal de fréquence instantanée est périodique, et sa période correspond à la période cardiaque fœtale.

Il s'agit donc d'estimer la période du signal de fréquence instantanée en observant ce signal sur les 1 à 5 secondes d'enregistrement pour en extraire son inverse : le RCF.

Selon d'autres exemples, d'autres approches sont retenues pour estimer le RCF :
- Une première approche est de prendre pour estimateur de la période du signal la valeur positive non nulle du temps qui maximise la fonction d'autocorrélation du signal de fréquence Doppler instantanée.
- Une seconde approche est de prendre pour estimateur du RCF la valeur de fréquence positive qui maximise le périodogramme du signal de fréquence Doppler instantanée.

### 6. Vérification des petits déplacements spatiaux du cœur

La séquence 5 peut être utilisée durant plusieurs minutes pour suivre le positionnement du cœur et au besoin pour optimiser ou recommencer l'optimisation du choix des capteurs du sous-groupe de capteurs du réseau qui tire.

### 7. Séquence alternative : positionnement cœur / mouvements fœtaux / mesure liquide amniotique

La séquence 3 est aussi utilisée pour cartographier les cavités de liquide amniotique. Les séquences 3 5 6 sont alternées pour optimiser le suivi du cœur fœtal. Une méthode de cartographie des cavités de liquide amniotique consiste à associer à chaque position de chaque faisceau ultrasonore des X transducteurs ultrasonores l'information de puissance du signal rétrodiffusée. Le liquide amniotique apparaît alors aux positions qui renvoient une puissance ultrasonore rétrodiffusée très faible par rapport aux autres tissus environnant.

Dans une variante, la séquence 3 est également utilisée pour cartographier les mouvements fœtaux. Une méthode de cartographie Doppler de ces mouvements consiste à d'abord filtrer dans le temps (c'est-à-dire suivant la séquence discrète des tirs successifs) les signaux ultrasonores démodulés en chaque position de chaque faisceau à travers un filtre passe-bande (dont la fréquence de coupure basse est de l'ordre de 30 Hz et la fréquence de coupure haute est de l'ordre de 90 Hz), puis à calculer la puissance moyenne de chacun de ces signaux en chaque point.

### 8. Détermination des déplacements / descente du fœtus en peripartum

Les séquences 5 et 6 sont alternées pour suivre le déplacement du fœtus durant sa descente.

### 9. Cartographie des mouvements et déplacements

La séquence 3 est utilisée pour cartographier les mouvements fœtaux, et caractériser ainsi le bien-être fœtal.

### 10. Multi-fœtus (au moins deux fœtus)

La séquence 3 est utilisée pour détecter plusieurs fœtus durant une grossesse multiple.

### 11. Doppler sur artère utérine, cordon ombilical

La séquence 3 est utilisée pour détecter des vaisseaux maternels ou le cordon ombilical et pour mesurer le flux sanguin correspondant. Elle permet de manière simultanée la mesure du rythme cardiaque maternel, et ainsi de distinguer rythme cardiaque fœtal et rythme cardiaque maternel.

### NOMENCLATURE

- 20: capteur ultrasonore
- 21: module de pilotage
- 22: module de traitement
- 23: tocomètre

## Revendications

1. Système électronique de surveillance fœtale, **caractérisé en ce qu'**il comprend :
- au moins quatre capteurs ultrasonores (20) émettant et réceptionnant des ondes ultrasonores de manière séquentielle, les capteurs ultrasonores (20) étant prévus pour être positionnés sur au moins une partie abdominale maternelle pour couvrir la structure cardiaque fœtale en mouvement et réaliser des mesures de fréquence cardiaque fœtale, la distance entre deux capteurs ultrasonores (20) est maintenue par une liaison, semi-rigide ou semi-élastique, afin de préserver la distribution géométrique des capteurs entre eux, la longueur de ladite liaison est comprise entre 30 et 60mm, de préférence entre 37 et 45 mm, le nombre de capteurs ultrasonores (20) forment ensemble un réseau triangulaire, carré, rectangulaire ou circulaire ;
- un module de pilotage (21) des capteurs ultrasonores (20) comprenant une information de commande des ondes ultrasonores envoyées à au moins un capteur ultrasonore (20) ;
- un module de traitement (22) des signaux ultrasonores pour estimer continûment la position du cœur, le rythme cardiaque fœtal et les mouvements fœtaux à partir du ou des signaux reçus des capteurs ultrasonores (20).

2. Système selon la revendication 1, dans lequel le nombre de capteurs ultrasonores (20) est compris entre 4 et 64, de préférence entre 24 et 32.

3. Système selon la revendication 1, dans lequel l'information de commande est un signal dont l'une de ces caractéristiques est prédéterminée par au moins l'un des éléments sélectionnés parmi : la phase, l'énergie, l'amplitude, la fréquence et la forme d'onde, la fréquence et la forme d'onde.

4. Système selon la revendication 3, dans lequel l'information de commande est indépendante d'un capteur ultrasonore (20) à un autre et en temps réel (20) à un autre et en temps réel.

5. Système selon la revendication 1, dans lequel le module de traitement (22) est relié par un élément de communication audit système, l'élément de communication étant filaire ou sans fil, l'élément de communication étant filaire ou sans fil.

6. Système selon la revendication 1, dans lequel la liaison sans fil utilise au moins une des modalités suivantes : les ondes hertziennes, par exemple UHF, lumineuses, par exemple infrarouges, sonores, par exemple infrasonores ou ultrasonores et/ou des spécifications de communication sur un réseau, par exemple Bluetooth, Wi-Fi, ou ZigBee.

7. Système selon la revendication 1, dans lequel il comporte un terminal communiquant configuré pour lire ou traiter les données du système.

8. Système selon la revendication 1, dans lequel il comporte l'un des éléments sélectionnés parmi : un tocodynamomètre, un capteur de saturation pulsée en oxygène (Sp02), un électrocardiogramme, un thermomètre, au moins un microphone, un accéléromètre, et un électromyogramme.

## Patentansprüche

1. Elektronisches System zur Fötusüberwachung, **dadurch gekennzeichnet, dass** es umfasst:
- mindestens vier Ultraschallsensoren (20), die sequentiell Ultraschallwellen senden und empfangen, wobei die Ultraschallsensoren (20) vorgesehen sind, auf mindestens einem Bauchabschnitt der Mutter positioniert zu sein, um die sich bewegende Herzstruktur des Fötus abzudecken und Herzfrequenzmessungen des Fötus durchzuführen, wobei der Abstand zwischen zwei Ultraschallsensoren (20) von einer halbstarren oder halbelastischen Verbindung gehalten wird, um die geometrische Verteilung der Sensoren untereinander zu bewahren, wobei die Länge der Verbindung zwischen 30 und 60 mm, vorzugsweise zwischen 37 und 45 mm, liegt, wobei die Anzahl der Ultraschallsensoren (20) gemeinsam ein dreieckiges, quadratisches, rechteckiges oder kreisförmiges Netz bilden;
- ein Steuermodul (21) der Ultraschallsensoren (20), umfassend eine Steuerinformation der an mindestens einen Ultraschallsensor (20) gesendeten Ultraschallwellen;
- ein Verarbeitungsmodul (22) der Ultraschallsignale, um die Position des Herzens, den Herzrhythmus des Fötus und die Bewegungen des Fötus auf der Basis des oder der von den Ultraschallsensoren (20) erhaltenen Signals/Signale kontinuierlich zu beurteilen.

2. System nach Anspruch 1, wobei die Anzahl der Ultraschallsensoren (20) zwischen 4 und 64, vorzugsweise zwischen 24 und 32, liegt.

3. System nach Anspruch 1, wobei die Steuerinformation ein Signal ist, von dem eines seiner Merkmale von mindestens einem der Elemente, ausgewählt aus der Phase, der Energie, der Amplitude, der Frequenz und der Form der Welle, der Frequenz und der Form der Welle, vorbestimmt ist.

4. System nach Anspruch 3, wobei die Steuerinformation von einem Ultraschallsensor (20) zu einem anderen unabhängig und in Echtzeit (20) zu einem anderen und in Echtzeit ist.

5. System nach Anspruch 1, wobei das Verarbeitungsmodul (22) durch ein Kommunikationselement mit dem System verbunden ist, wobei das Kommunikationselement drahtgebunden oder drahtlos ist, wobei das Kommunikationselement drahtgebunden oder drahtlos ist.

6. System nach Anspruch 1, wobei die drahtlose Verbindung mindestens eine der folgenden Modalitäten verwendet: die Hertzschen Wellen, beispielsweise UHF, die Lichtwellen, beispielsweise Infrarot, die Schallwellen, beispielsweise Infraschall oder Ultraschall und/oder die Netzwerkkommunikationsspezifikationen, beispielsweise Bluetooth, WLAN oder ZigBee.

7. System nach Anspruch 1, wobei es ein kommunizierendes Endgerät aufweist, das ausgelegt ist, um die Daten des Systems zu lesen oder zu verarbeiten.

8. System nach Anspruch 1, wobei es eines der Elemente aufweist, die aus einem Tokodynamometer, einem Pulssauerstoffsättigungssensor (SpO2), einem Elektrokardiogramm, einem Thermometer, mindestens einem Mikrofon, einem Beschleunigungsmesser und einem Elektromyogramm ausgewählt sind.

## Claims

1. An electronic system for foetal monitoring, **characterised in that** it comprises:
- at least four ultrasound sensors (20) sequentially emitting and receiving ultrasound waves, the ultrasound sensors (20) being provided to be positioned on at least one part of the mother's abdomen in order to cover the moving foetal cardiac structure and to carry out measurements of the foetal heart rate, the distance between two ultrasound sensors (20) is maintained by a semi-rigid or semi-elastic link, in order to preserve the geometric distribution of the sensors with each other, the length of said link is comprised between 30 and 60mm, preferably between 37 and 45 mm, the number of ultrasound sensors (20) together form a triangular, square, rectangular or circular network;
- a module (21) for controlling the ultrasound sensors (20) comprising a command information of the ultrasound waves sent to at least one ultrasound sensor (20);
- a module (22) for processing the ultrasound signals for continuously estimating the position of the heart, the foetal heart rhythm and the foetal movements from the signal(s) received from the ultrasound sensors (20).

2. The system according to claim 1, wherein the number of ultrasound sensors (20) is comprised between 4 and 64, preferably between 24 and 32.

3. The system according to claim 1, wherein the command information is a signal one of the features of which is predetermined by at least one of the elements selected from: phase, energy, amplitude, frequency and waveform, frequency and waveform.

4. The system according to claim 3, wherein the control information is independent from one ultrasound sensor (20) to another and in real time (20) to another and in real time.

5. The system according to claim 1, wherein the processing module (22) is linked by a communication element to said system, the communication element being wired or wireless, the communication element being wired or wireless.

6. The system according to claim 1, wherein the wireless link uses at least one of the following modalities: radio waves, for example UHF, light, for example infrared, sound, for example infrasonic or ultrasound waves and/or communication specifications on a network, for example Bluetooth, Wi-Fi, or ZigBee.

7. The system according to claim 1, wherein it includes a communicating terminal configured to read or process the data of the system.

8. The system according to claim 1, wherein it includes one of the elements selected from: a tocodynamometer, a pulsed oxygen saturation sensor (SpO2), an electrocardiogram, a thermometer, at least one microphone, an accelerometer, and an electromyogram.
